(19) **Europäisches Patentamt**

**European Patent Office**

**Office européen des brevets**

(11) Publication number: **0 422 396 B1**

## EUROPEAN PATENT SPECIFICATION

(45) Date of publication of patent specification: **13.12.95** (51) Int. Cl.⁶: **A61B 19/00**, G01R 33/58

(21) Application number: **90117489.6**

(22) Date of filing: **11.09.90**

---

(54) **Apparatus and method for calculating coordinate data of desired point in subject to be examined**

---

(30) Priority: **12.09.89 JP 236489/89**

(43) Date of publication of application:
**17.04.91 Bulletin 91/16**

(45) Publication of the grant of the patent:
**13.12.95 Bulletin 95/50**

(84) Designated Contracting States:
**DE NL**

(56) References cited:
**WO-A-88/00340**
**DE-A- 3 831 278**
**GB-A- 2 164 856**
**US-A- 4 644 276**

(73) Proprietor: **KABUSHIKI KAISHA TOSHIBA**
**72, Horikawa-cho,**
**Saiwai-ku**
**Kawasaki-shi,**
**Kanagawa-ken 210,**
**Tokyo (JP)**

(72) Inventor: **Machida, Yoshio, c/o Intellectual**
**Property Div.**
**Kabushiki Kaisha Toshiba,**
**1-1 Shibaura 1-chome**
**Minato-ku,**
**Tokyo 105 (JP)**

(74) Representative: **Blumbach, Kramer & Partner**
**Patentanwälte**
**Radeckestrasse 43**
**D-81245 München (DE)**

---

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to an apparatus for calculating coordinate data of a desired point in a subject to be examined by a computed tomographic apparatus such as an MRI apparatus (Magnetic Resonance Imaging Apparatus) according to the pre-characterizing part of claim 1, and a method of calculating coordinate data of a desired point in a subject to be examined by using the same.

Coordinate data of a desired point in a subject to be examined is used, for example, for stereotaxy surgery as one of operations of neurosurgeon. In stereotaxy, the position of a morbid portion in the brain of a patient is identified with the aid of an image, and an operation is performed, e.g., inserting a biopsy cannula in the identified portion so as to remove the morbid portion. As the image, an X-ray image (Roentgen image) obtained by an X-ray diagnosing apparatus has been used. Recently, however, CT stereotaxy using an X-ray CT apparatus as a computed tomographic apparatus and MRI stereotaxy using an MRI apparatus as a computed tomographic apparatus have been performed.

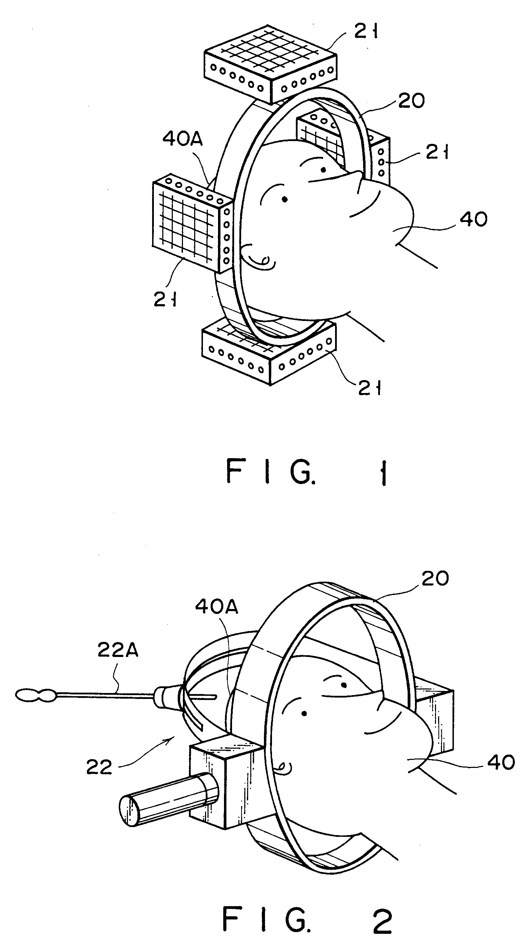

MRI stereotaxy is conventionally realized by the coordinate calculation step of calculating the coordinate value of a morbid portion in a subject to be examined, and the step of operating a stereotactic apparatus on the basis of the calculated coordinate value, i.e., the operation step. Fig. 1 shows an apparatus used in the coordinate calculation step and a subject to be examined. Fig. 2 shows an apparatus used in the operation step, i.e., a stereotactic apparatus and the subject.

As shown in Fig. 1, in the apparatus used in the coordinate calculation step, a head portion 40A of a subject 40 to be examined is held in an annular frame 20. In this case, an indicator phantom 21 is mounted on the frame 20 in order to indicate X-, Y-, and Z-coordinates. The frame 20 is placed at a predetermined position in a photographable region of a computed tomographic apparatus (not shown) while the head portion 40A is held by the frame 20, and a photographing operation is performed to obtain, e.g., an axial plane image. By observing this image, the position of the morbid portion appearing on the image is recognized and identified with X-, Y-, and Z-coordinates by referring to the indicator phantom 21. The indicator phantom 21 is detached from the frame 20, and a stereotactic apparatus 22 is mounted. Thereafter, for example, a biopsy cannula 22A of the apparatus 22 is pierced into the head portion 40A to reach the position coordinates of the morbid portion obtained in the above-described step as a target, and the morbid portion is removed.

In the execution of the above-described stereotaxy, since a surgical operation is to be performed for a brain, high positional precision is required in identification of a morbid portion.

GB-A-2 164 856 discloses an apparatus in accordance with the pre-characterizing part of claim 1 and also in accordance with the pre-characterizing part of claim 19. The frame to be attached to the subject includes a plurality of positioning means which are intended to ensure that when the frame is repeatedly attached to the subject, the relationship between the frame and the subject is always the same. In order to carry out stereotaxy surgery, a stereotaxy apparatus is attached to the frame so that during the operation using the stereotaxy apparatus the coordinates obtained by an indicator phantom can be used as a reference for obtaining coordinate values.

As mentioned above, MRI stereotaxy includes a coordinate calculation step of calculating the coordinate values of a morbid in a subject.

In an MRI apparatus, however, because of the characteristics of static and gradient fields, field distortion is abruptly increased with an increase in distance from the field center, and following this field distortion, image distortion is abruptly increased. Example, image distortion corresponding to 5 mm may be caused at a position near the frame, thus posing a problem in execution of stereotaxy requiring high positional precision.

US-A-4 644 276 discloses a coordinate phantom to be used in an MRI apparatus in order to obtain calibration data, the phantom comprising a plurality of test plates.

It is the object of the present invention to provide an apparatus for calculating coordinate data of a desired point in a subject to be examined and a method of calculating coordinate data of a desired point in a subject to be examined by using the same, more particularly, an apparatus and method capable of obtaining high-precision coordinate data.

This object is achieved according to the invention by the features of claims 1, 5, 12 and 19, respectively.

According to the above-described apparatuses and methods, since almost no image distortion is present inside the frame placed in the photographable region of the computed tomographic apparatus, a frame coordinate phantom image appearing on a phantom image represents the positional coordinates of the frame. Therefore, a correct position of the frame itself and a correct positional relationship between the frame and a subject to be examined can be obtained on the basis of the phantom image and a subject image photographed at the same position as that assumed when the phantom image is photographed. With this, calibration data for calibrating the deviation between the coordinates of the subject on the real space and the coordinates of the subject on the image photographed by the computed tomographic apparatus can be obtained.

In addition, since almost no image distortion is present inside the frame placed in the photographable region in the computed tomographic apparatus, a frame coordinate phantom image appearing on a phantom image represents the positional coordinates of the frame. Therefore, a correct position of the frame itself and a correct positional relationship between the frame and a subject to be examined can be obtained on the basis of the phantom image and a subject image photographed at the same position as that assumed when the phantom image is photographed. With this, calibration data for calibrating the deviation between the coordinates of the subject on the real space and the coordinates of the subject on the image photographed by the computed tomographic apparatus can be obtained. Moreover, since both the images are obtained at the frame having the indicator phantom mounted on the outer surface thereof, a more correct positional relationship of the frame between the images can be obtained.

This invention can be more fully understood from the following detailed description when taken in conjunction with the accompanying drawings, in which:

Fig. 1 is a perspective view showing a conventional coordinate calculation apparatus constituted by a frame and indicator phantoms in relation to a subject to be examined;

Fig. 2 is a perspective view showing a conventional stereotactic apparatus in relation to a frame and a subject to be examined;

Fig. 3 is a view showing an overall arrangement of an MRI apparatus used in the present invention;

Figs. 4 to 8 show problems based on the field characteristics of the MRI apparatus, in which Fig. 4 is a graph showing static field distortion characteristics,

Fig. 5 is a graph showing gradient field distortion characteristics,

Fig. 6 is a graph showing distortion characteristics based on static and gradient fields,

Fig. 7 is a graph showing image distortion appearing when static field distortion is present, and

Fig. 8 is a graph showing image distortion appearing when gradient field distortion is present;

Figs. 9A and 9B show a coordinate calculation apparatus according to the first embodiment of the present invention, which includes indicator phantoms and a detachable frame coordinate phantom, in which

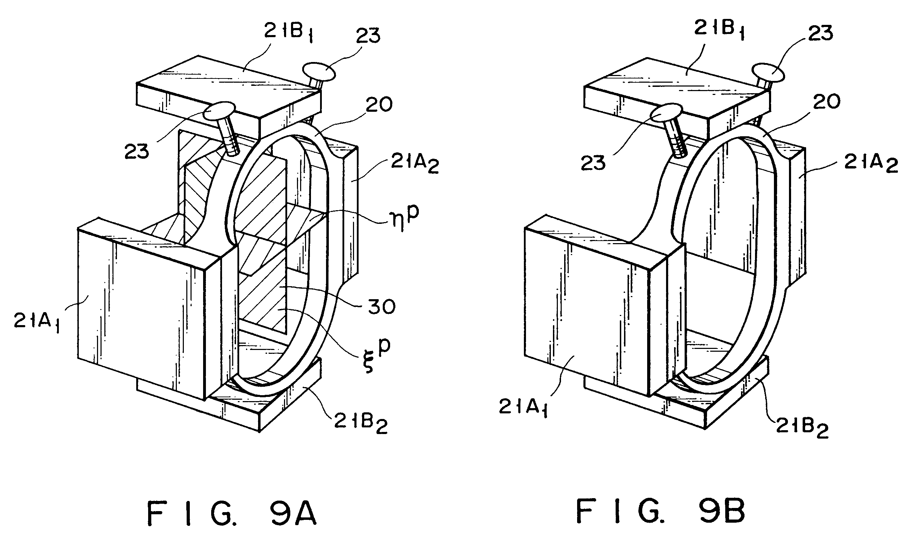

Fig. 9A is a perspective view showing a state wherein phantom images are to be photographed, and

Fig. 9B is a perspective view showing a state wherein a subject image is to be photographed;

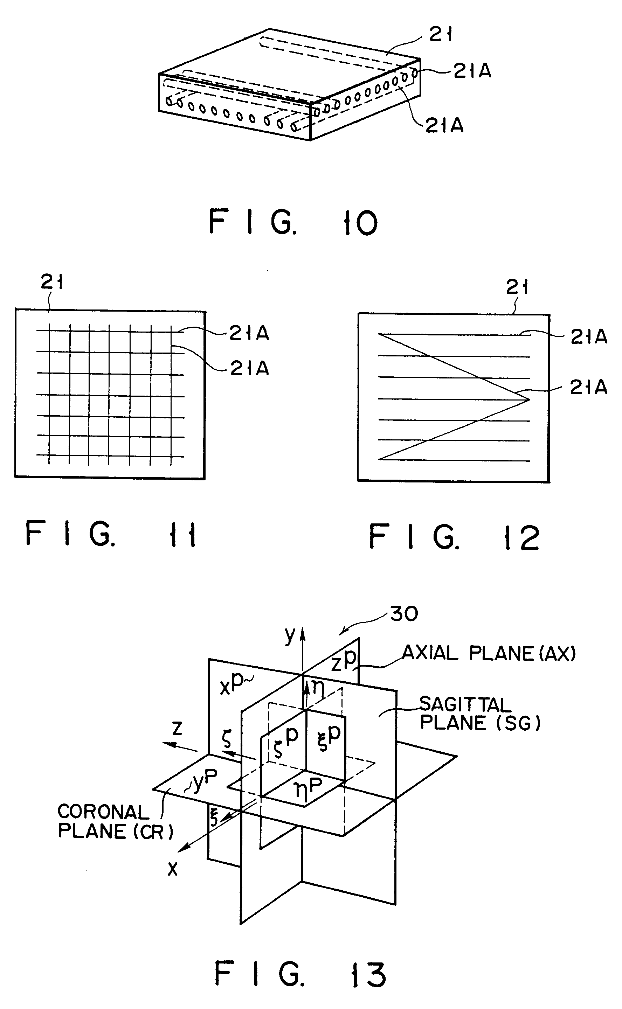

Fig. 10 is a perspective view showing an indicator phantom as an example;

Fig. 11 is a plan view showing an indicator phantom as another example;

Fig. 12 is a plan view showing an indicator phantom as still another example;

Fig. 13 is a perspective view showing a MRI apparatus coordinate system and a frame (the coordinate calculation apparatus) coordinate system as an example;

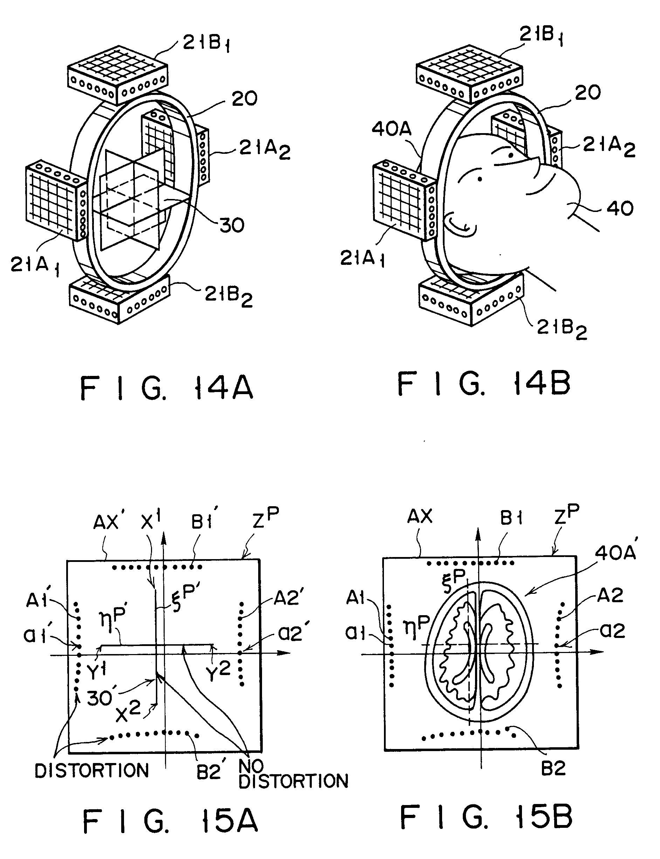

Fig. 14A is a perspective view showing a state wherein phantom images are to be photographed by using the coordinate calculation apparatus in Fig. 9A;

Fig. 14B is a perspective view showing a state wherein a subject image is to be photographed by using the coordinate calculation apparatus in Fig. 9B;

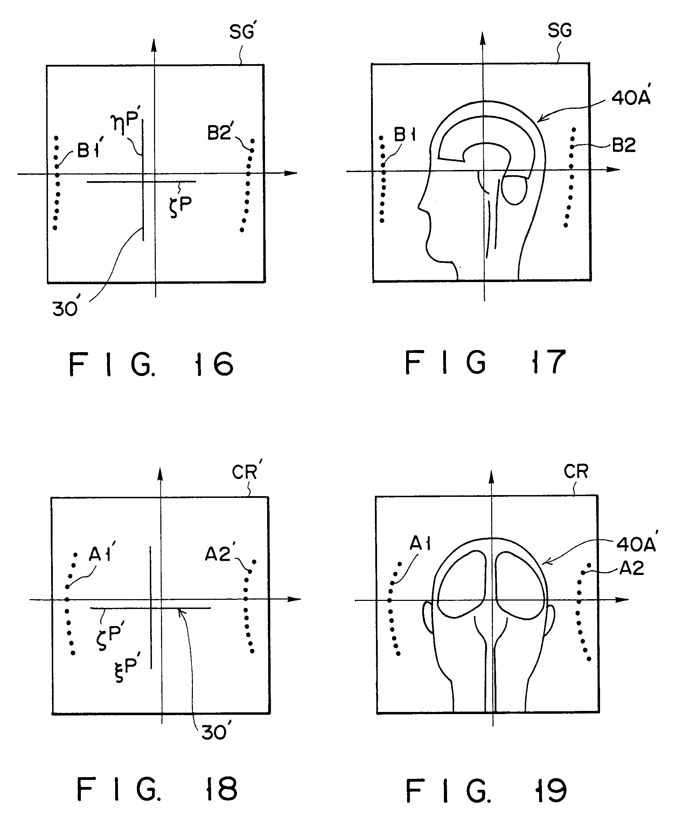

Fig. 15A is a view showing phantom images on an axial plane by using the apparatus in Fig. 14A;

Fig. 15B is a view showing a subject image on an axial plane by using the apparatus in Fig. 14A;

Figs. 16 and 17 are views showing a case wherein coordinate calibration is performed by applying the present invention to images on sagittal planes;

Figs. 18 and 19 are views showing a case wherein coordinate calibration is performed by applying the present invention to images on coronal planes;

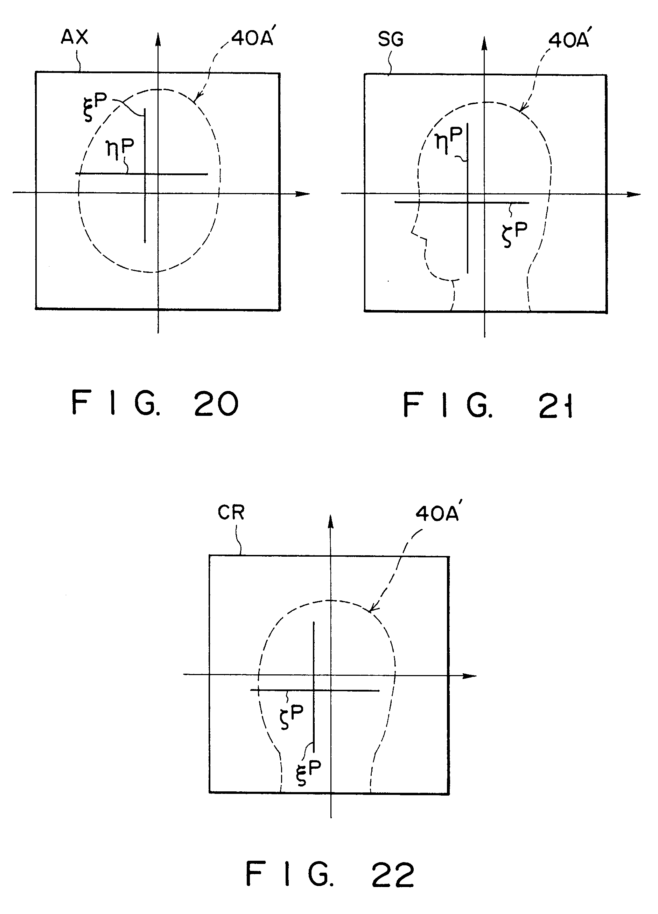

Fig. 20 is a view showing a case wherein coordinate calibration is performed by applying the present invention to phantom images;

Fig. 21 is a view showing a case wherein coordinate calibration is performed by applying the present invention to images on a sagittal plane;

Fig. 22 is a view showing a case wherein coordinate calibration is performed by applying the present invention to images on a coronal plane;

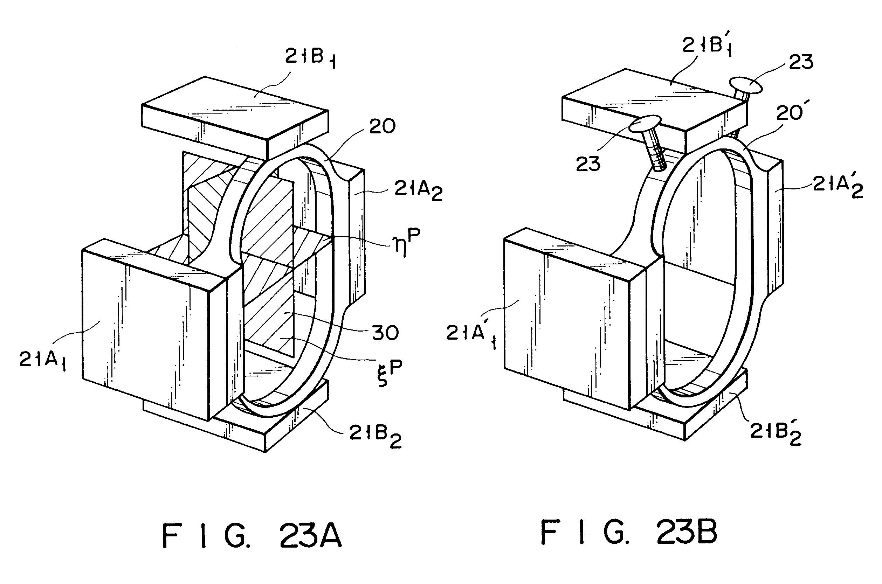

Figs. 23A and 23B show a coordinate calculation apparatus according to the second embodiment of the present invention, which includes indicator phantoms and a fixed type frame coordinate phantom, in which

Fig. 23A is a perspective view showing a state wherein phantom images are to be photographed, and

Fig. 23B is a perspective view showing a state wherein a subject image is to be photographed;

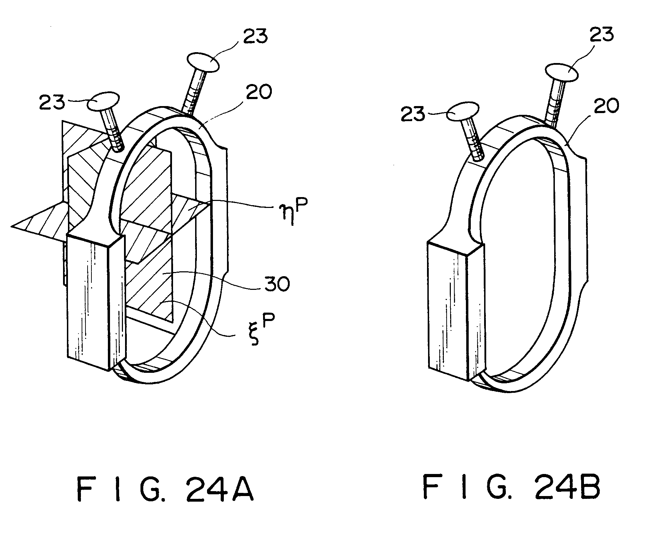

Figs. 24A and 24B show a coordinate calculation apparatus according to the third embodiment of the present invention, which does not include any indicator phantom but includes a detachable frame coordinate phantom, in which

Fig. 24A is a perspective view showing a state wherein phantom images are to be photographed, and

Fig. 24B is a perspective view showing a state wherein a subject image is to be photographed;

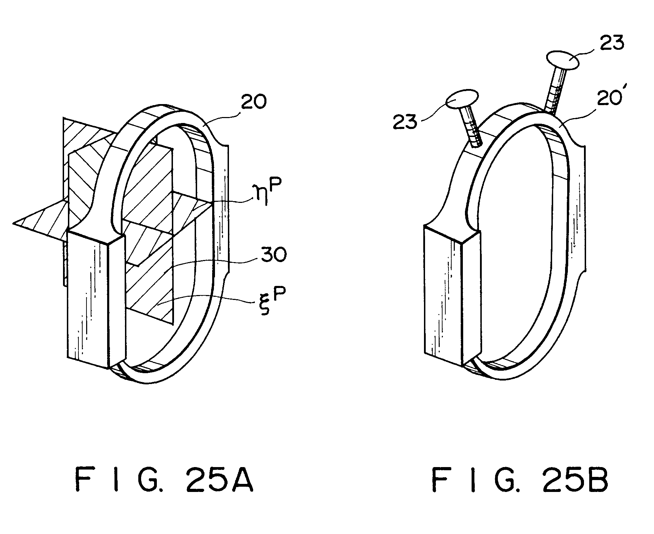

Figs. 25A and 25B show a coordinate calculation apparatus according to the fourth embodiment of the present invention, which does not include any indicator phantom but includes a fixed type frame coordinate phantom, in which

Fig. 25A is a perspective view showing a state wherein phantom images are to be photographed, and

Fig. 25B is a perspective view showing a state wherein a subject image is to be photographed;

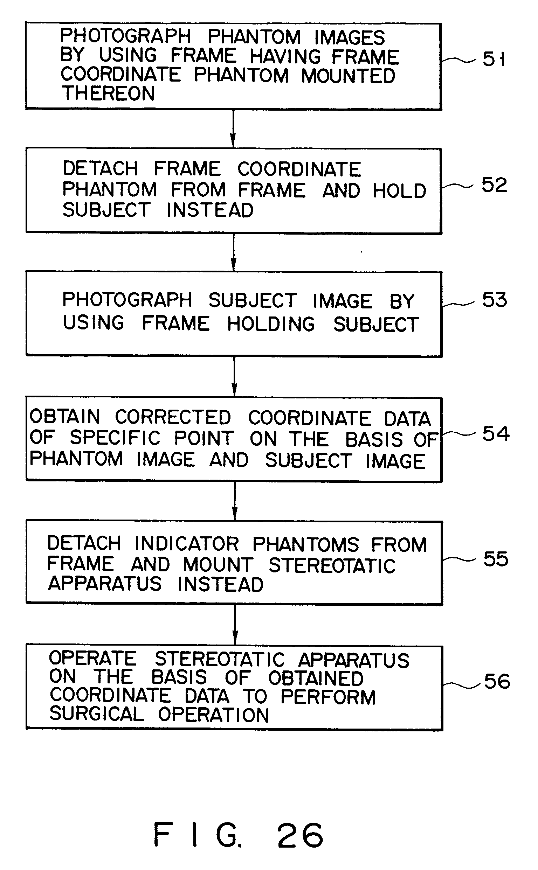

Fig. 26 is a flow chart showing a method of performing coordinate calibration and stereotaxy by using the coordinate calculation apparatuses in Figs. 9A and 9B and Figs. 24A and 24B; and

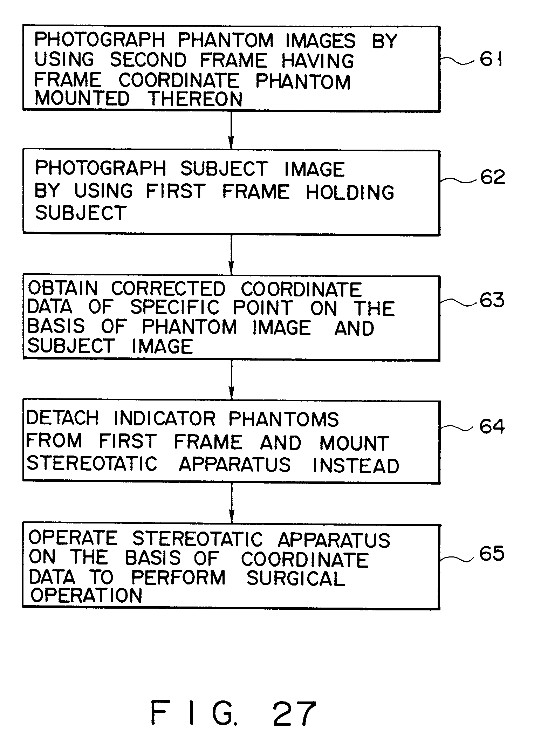

Fig. 27 is a flow chart showing a method of performing coordinate calibration and stereotaxy by using the coordinate calculation apparatuses in Figs. 23A and 23B and Figs. 25A and 25B.

An MRI apparatus will be described below.

An MR phenomenon is a phenomenon in which an atomic nucleus having a non-zero spin and a magnetic moment and placed in a static field resonantly absorbs/radiates an electromagnetic wave having a specific frequency. The atomic nucleus resonates at an angular frequency $\omega_0$ ($\omega_0 = 2\pi\gamma_0$, $\gamma_0$; Larmor frequency) given by the following equation:

$$\omega_0 = \gamma H_0$$

where $\gamma$ is the gyromagnetic ratio unique to the type of atomic nucleus, and $H_0$ is the static field intensity.

The apparatus for diagnosing a living body by using the above-mentioned principle is designed to obtain diagnosis data reflecting an atomic nucleus density, a longitudinal relaxation time $T_1$, a transverse relaxation time $T_2$, a flow, a chemical shift, and the like, e.g., a slice image of a subject to be examined in a nondestructive manner by performing signal processing of an electromagnetic wave having the same frequency as described above which is excited upon the above-mentioned resonance absorption.

In acquisition of diagnosis data by MR, the entire portion of the subject placed in the static field can be excited and signals therefrom can be acquired. In practice, however, an MRI apparatus is designed to excite a specific portion and acquire signals therefrom due to limitations on the apparatus arrangement and clinical requirements in terms of imaging.

In this case, a specific portion as an imaging target is generally a slice portion having a certain thickness. Echo signals or MR signals of FID signals from this slice portion are acquired by executing a data encode procedure a large number of times. The acquired data group is then subjected to image reconstruction processing by a two-dimensional Fourier transform method, thus generating an image of the specific slice portion.

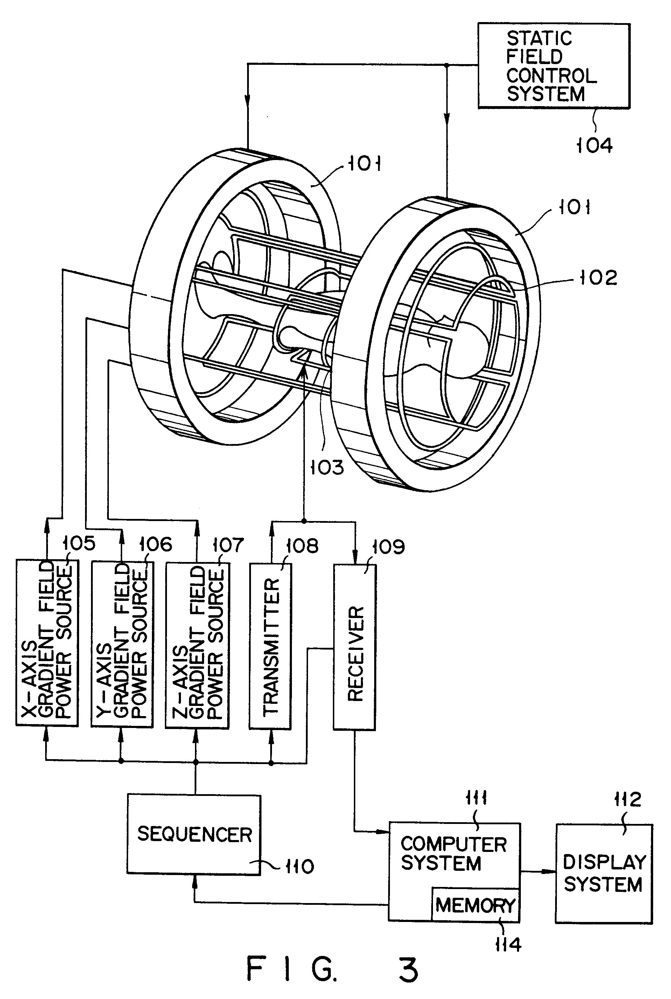

Fig. 3 shows an overall arrangement of an MRI apparatus used in the present invention. As shown in Fig. 3, a magnet assembly which can accommodate a subject 40 to be examined includes static coils 101 of a normal conduct ion or superconduction scheme (may be constituted by permanent magnets) 101, X-, Y-, and Z-axis gradient field generating coils 102 for generating gradient fields to provide position data of an excited portion to an MR signal, and a probe 103 constituted by a transmission/reception system, e.g., transmission and reception coils, for transmitting a rotating high-frequency field and detecting excited MR signals (echo signals and FID signals).

If the superconduction scheme is employed, the apparatus includes a supply control system for a coolant and is mainly constituted by a static field control system 104 for ON/OFF-controlling a static field power source, a transmitter 108 for performing transmission control of RF pulses, a receiver 109 for performing reception control of excited MR signals, X-, Y-, and Z-axis gradient field power sources 105, 106, and 107 for performing excitation control of the X-, Y-, and Z-axis gradient field generating coils 102, a sequencer 110 for executing a pulse sequence for data acquisition, a computer system 111 for controlling the respective components and performing signal processing of a detection signal, and a display unit 112 for displaying an image based on the processed signal. In addition, the computer system 111 includes a memory 114 for storing a phantom image (to be described later).

In the pulse sequence for data acquisition, the transmitter 108 is driven to apply an RF pulse of a rotating field from the probe 103 to the subject 40. At the same time, the gradient field sources 105, 106, and 107 are driven to apply gradient fields $G_X$, $G_Y$, and $G_Z$ as a slice gradient field ($G_S$), a phase encode gradient field ($G_E$), and a read gradient field ($G_R$), respectively, from the gradient field generating coils 102 to the subject 40. With this operation, a signal from a specific portion is acquired by the probe 3. A data group is obtained by repeatedly executing this sequence a predetermined number of times, and an image is generated from this data group.

An MRI apparatus of this type which has recently been regarded as a promising apparatus has the following advantages over an X-ray CT apparatus (X-ray computed tomographic apparatus) which is often compared with the former apparatus. In the MRI apparatus, an arbitrary tomographic image of a subject (to be examined) such as an axial plane image, a sagittal plane image, a coronal plane image, an oblique plane image can be obtained by a simple electrical operation.

In addition, a photographed image is diagnosis data reflecting an atomic nucleus density, spin-lattice relaxation times $T_1$ and $T_2$, a flow, a chemical shift, and the like, and hence is expected to be used in a variety of manners in image diagnosis. For example, in the MRI apparatus, the structure of a lower portion of a deep-brain can be imaged without any artifact, and a minute nuclei-nervus can also be imaged.

With the above advantages, the use of the MRI apparatus has been studied and effectively practiced in image diagnosis which cannot be appropriately performed by the X-ray CT apparatus, especially in the field of diagnosis of neurosurgeon.

The MRI apparatus as a computed tomographic apparatus, however, has the following characteristics, which pose problems in terms of accuracy of identification, i.e., proper execution of stereotaxy. In the MRI apparatus, static and gradient fields are distorted. For this reason, an actual object (an object on an actual space) does not properly appear on an acquired image in relation to the shape and position of the object.

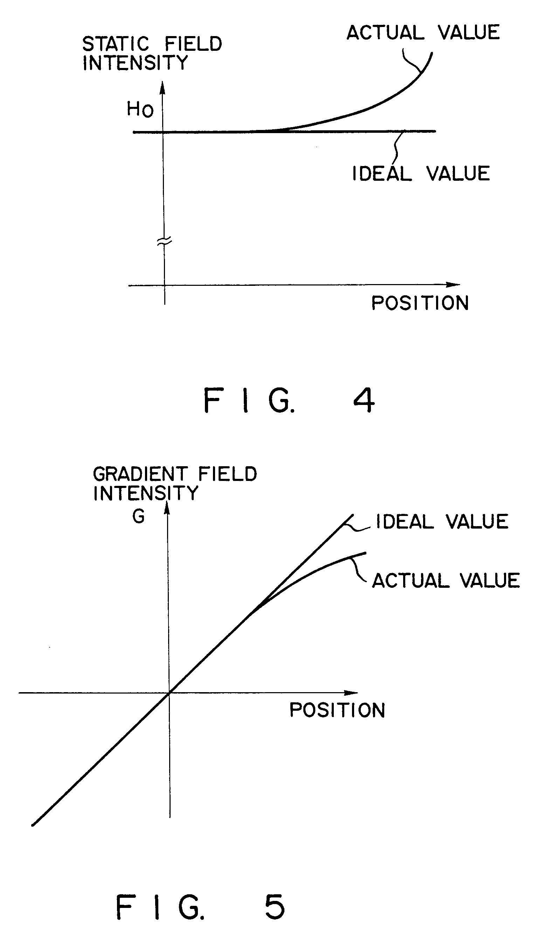

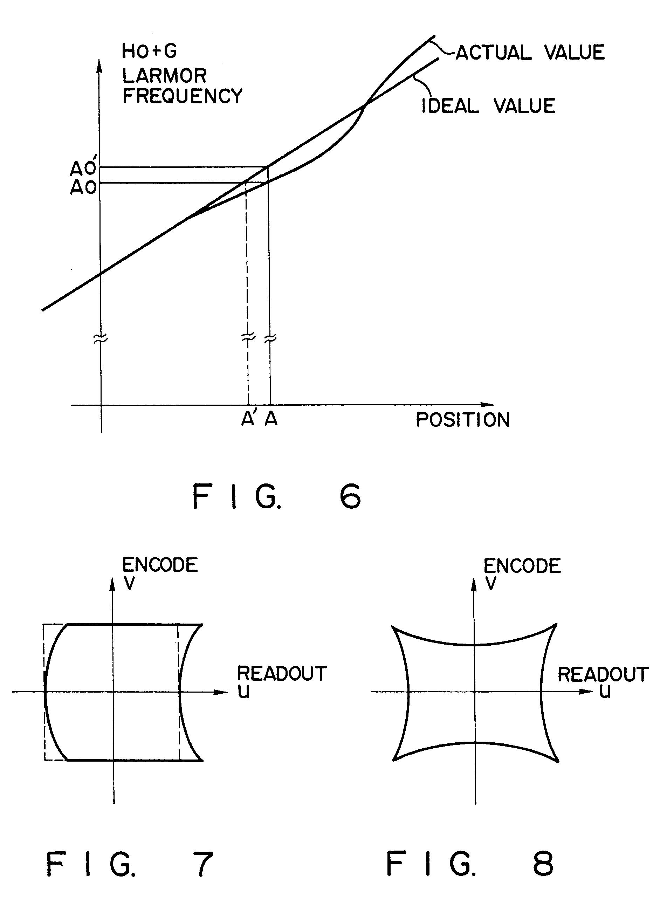

That is, as shown in Fig. 4, the ideal static field intensity H0 is spatially uniform, but an actual intensity is distorted. In addition, as shown in Fig. 5, an ideal gradient field intensity G spatially has linearity, but an actual intensity is distorted. Therefore, an acquired image inevitably includes a positional deviation. As shown in Fig. 6, a gradient field intensity corresponding to static field intensity $H_0$ + gradient field intensity G = Larmor frequency ideally exhibits linear characteristics, but is actually distorted.

For this reason, a field intensity at a point A in Fig. 6 is ideally a field intensity $A_0$ but is actually a field intensity $A_0'$. In addition, the field intensity $A_0'$ should appear at a point A' but actually appears at a different point, which is similar to the presence of a real image A and an actual image A'. Moreover, the distortion of the static field intensity $H_0$ and of the gradient field intensity G is abruptly increased with an increase in distance from the field center, thus posing a problem.

Fig. 7 illustrates a state wherein the static field intensity $H_0$ has nonuniformity. In this case an image of an object indicated by a dotted line (square) is distorted in a direction $\underline{u}$ of the read gradient field $G_R$.

Fig. 8 illustrates a state wherein the gradient field intensity G has nonlinearity. In this case, the image of the object indicated by the dotted line (square) is distorted in the direction $\underline{u}$ of the read gradient field $G_R$ and a direction $\underline{v}$ of the encode gradient field $G_E$.

As described above, in the MRI apparatus, distortion is abruptly increased with an increase in distance from the field center because of the characteristics of static and gradient fields. Distortion corresponding to about 5 mm may occur at a position near a frame. This poses a serious problem in the execution of stereotaxy requiring high positional precision.

Under the circumstances, the apparatus and method of the present invention which will be described in detail below are effective.

An embodiment of coordinate calculation according to the present invention will be described below with reference to the accompanying drawings.

Figs. 9A and 9B are perspective views showing a stereotaxy frame, an indicator phantom, and a frame coordinate phantom which constitute a main portion of a coordinate calculation apparatus according embodiment of the present invention.

As shown in Fig. 9A, a stereotaxy frame 20 can be arranged in a photographable region in the gantry of an MRI apparatus (not shown) and is designed to hold a head portion 40A of a subject 40 inside its annular frame. The frame 20 includes a plurality of screws 23 for fixing the head portion 40A.

In addition, four indicator phantoms 21 (21AI, 21A2, 21BI, and 21B2) each of which can be photographed by the MRI apparatus and has an X-Y-Z coordinate display member are arranged outside the frame 20. These indicator phantoms 21 are detachably mounted on the frame 20.

Figs. 10 to 12 show examples of the indicator phantom 21. As shown in Figs. 10 and 11, the indicator phantom 21 may be designed such that a plurality of water pipes 21A are arranged parallel to each other, and a plurality of other water pipes 21A are arranged parallel to each other on the former pipes 21A so as to be perpendicular to each other. Alternatively, as shown in Fig. 12, the indicator phantom 21 may be designed such that a plurality of water pipes 21A are arranged parallel to each other, and two other water pipes 21A are obliquely arranged on the former wate pipes 21A. With such an arrangement, three-axis orthogonal coordinates can be recognized by an image of the water pipes 21A appearing on an acquired tomographic image.

A frame coordinate phantom 30 is detachably arranged inside the frame 20. This frame coordinate phantom 30 can be photographed by the MRI apparatus and has an X-Y-Z coordinate display member constituted by, e.g., three orthogonal planes. With the above-described arrangement, for example, the stereotactic apparatus 22 shown in Fig. 2 can be mounted on the frame 20 after the indicator phantoms 21 are detached from the frame 20.

A coordinate calculation method using the coordinate calculation apparatus of this embodiment, which has the above-described arrangement, will be described below together with a description of MRI stereotaxy.

The coordinate system will be described first. Assume, as shown in Fig. 13, that the MRI apparatus has the X-Y-Z coordinate system, and the coordinate calculation apparatus (frame 20) has the $\xi$-$\eta$-$\zeta$ coordinatesystem. With this arrangement, an image for calibration (phantom image) is given by the X-Y-Z coordinate system. In addition, the planes of the frame coordinate system are respec-tively represented by the $\xi$-$\eta$, $\eta$-$\zeta$ and $\zeta$-$\xi$ planes. In this case, the $\xi$-$\eta$ plane is represented by $\zeta^P$; the $\eta$-$\zeta$ plane, by $\xi^P$; and $\zeta$-$\xi$ plane, by $\eta^P$.

As is shown in Fig. 14A, the indicator phantoms 21 are mounted on the surface of the frame 20. A frame coordinate phantom 30 is located in the frame 20. The frame 20, the indicator phantoms 21, and the frame coordinate phantom 30 constitute an integral unit. Preferably, the phantom 30 is fixed to the frame 20 at the position where the stereotaxy device is attached. This is because the stereotaxy device has a high mechanical precision. The frame 20 is positioned within the gantry of the MRI apparatus, such that the integral unit is placed in the photographable region in the gantry, preferably at the position where the intersection of the phantom 30 is located at the center of the magnetic field. When a slice photographing operation is performed along, e.g., the X-Y plane, a phantom image AX′ of an axial plane image or the like on which images (A1′, A2′, B1′, and B2′) of the indicator phantoms 21 and an image 30′ ($\zeta$P, $\eta$P) of the frame coordinate phantom 30 appear is acquired, as shown in Fig. 15A.

Subsequently, the frame coordinate phantom 30 is detached from the frame 20. As shown in Fig. 14B, the head portion 40A of the subject 40 is held in place of the frame coordinate phantom 30, and the frame 20 which is holding the head portion 40A is fixed to the same position as that assumed when the phantom image is photographed, which is located in the photographable region of the MRI apparatus, upon positioning control. Thereafter, similar to the above-described operation of acquiring the phantom image, a slice photographing operation is performed along, e.g., the X-Y plane to acquire, e.g., a subject image AX of an axial plane image on which an image (40A′) of the head portion 40A and images (Al, A2, B1, and B2) of the indicator phantoms 21 appear.

These phantom and subject images are used to obtain calibration data for calibrating the deviation between the position (coordinates) of the frame 20 and the position (coordinates) of the head portion 40A on the real space. The subject image is observed to recognize a desired position on the image as a morbid portion, and the coordinates of the recognized position on the real space are calculated by using the calibration data. This calculation method is realized by data processing by means of a computer or the like, which will be described in detail later.

Subsequently, the indicator phantoms 21 are detached from the frame 20, and, for example, the stereotactic apparatus 22 shown in Fig. 2 is mounted on the frame 20 while the head portion 40A is held therein. The biopsy cannula 22A is inserted in the head portion 40A to the position (coordinates) of the morbid portion obtained by the above-mentioned processing as a target so as to remove the morbid portion.

The calculation method of recognizing the deviation between the position (coordinates) of the frame 20 and the position (coordinates) of the head portion 40A on the real space by using the phantom and subject images, i.e., obtaining the calibration data will be described below.

The images (A1′, A2′, B1′, and B2′) of the indicator phantoms 21 on the phantom image AX′ and the images (Al, A2, Bl, and B2) of the indicator phantoms 21 on the subject image AX respectively appear at substantially the same positions. By using this phenomenon, a transformation formula $\phi$- ($\phi_{AX}(AX') \cong AX$) for causing these images to coincide with each other is obtained. Since the image 30′ of the frame coordinate phantom 30 represents coordinates inside the frame 20, a position (coordinates) on the real space can be obtained on the basis of the transformation formula and the coordinates inside the frame 20.

If, for example, a target point is set on the subject image AX, the coordinates of the target position can be recognized from the image 30′ of the frame coordinate phantom image 30. The obtained coordinates are compensated by the trans-formation formula $\phi$. Therefore, the biopsy cannula 22A can be properly inserted in a portion corresponding to the position data of the head portion 40A on the real space. Note that the transformation formula $\phi$ is calculated by two dimension congruent transformation of superimposing using at least two points of the group of Al′, A2′, Bl′, and B2′ and the group of Al, A2, Bl, and B2.

The calculation of the transformation formula for causing the images (Al′, A2′, Bl′, and B2′) of the indicator phantoms 21 and the images (Al, A2, B1, and B2) of the indicator phantoms 21 on the subject image AX to coincide with each other is a means for ensuring positional coincidence between the phantom image AX' and the subject image AX. If, for example, high-precision bed date control is performed and satisfactory reproducibility can be obtained in relation to photography positioning, position data may be obtained simply on the basis of the image 30' of the frame coordinate phantom and the image 40A; of the head portion 40 without calculation of the transformation formula $\phi$.

Figs. 15A and 15B show the case wherein the present invention is applied to images on axial planes. However, the present invention is not limited to this. For example, the present invention can be applied to images on sagittal planes as shown in Fig. 16 and 17 and can also be applied to images on coronal planes as shown in Figs. 18 and 19 in the same manner as described above.

In addition, a phantom image and a plurality of subject images may be used. For example, Figs. 20 to 22 show a case wherein the present invention is applied to a phantom image, an image on a sagittal plane, and an image on a coronal plane.

Further, the transformation formula $\phi$ can be changed to a three-dimensional transformation formula $\phi'$ by performing calibration in three directions, and then more accurate position data can be acquired by solving the three-dimensional transformation formula $\phi'$.

In the above-described case, each indicator phantom and the frame coordinate phantom need not have threeaxis coordinate display members but may have two-axis coordinate display members capable of specifying positions.

Figs. 23A and 23B are perspective views showing a coordinate calculation apparatus according to the second embodiment of the present invention, which includes indicator phantoms 21 and a fixed type frame coordinate phantom 30.

Fig. 23A shows a state wherein phantom images are photographed. Fig. 23B shows a state wherein a subject image is to be photographed.

In the first embodiment, the frame coordinate phantom 30 is detachably mounted on the single frame 20. In contrast to this, in the second embodiment, in addition to a frame 20 to which the frame coordinate phantom 30 is fixed, another frame 20' including the indicator phantoms 21 is used. This frame 20' has geometrically the same shape as that of the frame 20. A subject image is photographed by using this frame 20', whereas phantom images are photographed by using the frame 20. That is, phantom and subject images are photographed by using the different frames 20 and 20'.

Fig. 24A and 24B are perspective views showing a coordinate calculation apparatus according to the third embodiment of the present invention, which does not include the indicator phantoms 21 but includes a detachable frame coordinate phantom 30.

Fig. 24A shows a state wherein phantom images are to be photographed. Fig. 24B shows a case wherein a subject image is to be photographed. That is, phantom and subject images are photographed by using the same frame 20.

The apparatus of the third embodiment ifs identical to the apparatus of the first embodiment from which the indicator phantoms 21 are omitted.

Figs. 25A and 25B are perspective views showing a coordinate calculation apparatus according to the fourth embodiment of the present invention, which does not include the indicator phantoms 21 but includes a fixed type frame coordinate phantom 30.

Fig. 25A shows a state wherein phantom images are to be photographed. Fig. 25B shows a state wherein a subject image is to be photographed.

The apparatus of the fourth embodiment is identical to the apparatus of the second embodiment from which the indicator phantoms 21 are omitted. That is, phantom and subject images are photographed by using different frames 20 and 20'.

Fig. 26 is a flow chart showing a routine constituted by steps 51, 52, 53, 54, 55, and 56, which shows a method of performing coordinate calibration and stereotaxy by using the coordinate calculation apparatuses shown in Figs. 9A and 9B and Figs. 24A and 24B. This routine is applied to a case wherein phantom and subject images are photographed by using the same frame 20. In this case, step 51 includes a procedure for controlling of a reference frequency corresponding a static field intensity of changing with passage of time in the MRI apparatus, thereby to improve the precision. A reference frequency (i.e., Larmor frequency) is adjusted in accordance with the intensity of the static magnetic field which changes with time. This is a so-called "magnetic field-locking control," in which the Larmor frequency $\omega$ is adjusted so as to satisfy Larmor equation $\omega = H_0$, where $H_0$ is the intensity of the static field which slightly changes with time, and $\omega$ is Larmor frequency $\omega$ preset on hardware. In addition, photography conditions in step 51 are the same as those in step 52. This also contributes to improvement in precision. In this case, photography conditions in the MRI apparatus are conditions including a pulse sequence and influencing the quality of a generated image.

Fig. 27 is a flow chart showing a routine constituted by steps 61, 62, 63, 64, and 65, which shows a method of performing coordinate calibration and stereotaxy by using the coordinate calculation apparatuses shown in Figs. 23A and 23B and Figs. 25A and 25B. This routine is applied to a case wherein phantom and subject images are photographed by using the different frames 20 and 20'. In this case, step 61 includes a procedure for controlling of a reference frequency corresponding a static field intensity of changing with passage of time in the MRI apparatus. This procedure is designed to improve the precision. In addition, photography conditions in step 61 are the same as those in step 62. This also contributes to improvement in precision.

In addition, one phantom image can be stored in the memory 114 of the computer system 111. This saves photographing a phantom image for each subject to be examined.

As has been described above, according to the apparatus and method of the present invention, image distortion caused in an MRI apparatus can be compensated, and high-precision stereotaxy can be executed.

## Claims

1. An apparatus for calculating coordinate data of a desired point in a subject (40A) to be examined by using a frame (20') which has a shape capable of holding the subject (40A) therein and can be arranged in a photographable region in a gantry of a computed tomographic apparatus,
characterized by comprising:
another frame (20) having geometrically the same shape as that of said frame (20'); and
a frame coordinate phantom (30) which is fixed inside said another frame (20), has a coordinate display member capable of displaying coordinates of at least one axis of X-, Y-, and Z-coordinates, and can be photographed by said computed tomographic apparatus.

2. An apparatus according to claim 1, characterized in that said computed tomographic apparatus is a magnetic resonance imaging apparatus.

3. An apparatus according to claim 1, characterized in that said frame (20') allows a stereotactic apparatus (22) to be detachably mounted thereon.

4. An apparatus according to claim 1, 2 or 3, comprising an indicator phantom ($21A_{1'}$, $21A_{2'}$, $21B_{1'}$, $21B_{2'}$) which is fixed to an outer surface of said frame (20') has a coordinate display member capable of displaying coordinates of at least one axis of X-, Y-, and Z-coordinates, and can be photographed by said computed tomographic apparatus, and
another indicator phantom ($21A_{1'}$, $21A_{2'}$, $21B_{1'}$, $21B_{2'}$) which is fixed to an outer surface of said another frame (20), has a coordinate display member capable of displaying coordinates of at least one axis of X-, Y-, and Z-coordinates, and can be photographed by said computed tomographic apparatus.

5. A method of calculating coordinate data of a desired point in a subject to be examined by using said apparatus of claim 1, comprising the steps of:
arranging said another frame (20) having said frame coordinate phantom (30) fixed thereto in a photographable region in a computed tomographic apparatus, and integrally photographing said another frame (20) and said frame coordinate phantom (30), thereby obtaining a phantom image (61);
arranging said frame (20') holding the subject therein at the same position as that assumed when the phantom image is photographed, and integrally photographing said frame (20') and the subject (40A), thereby obtaining a subject image (62);
obtaining a positional relationship between said frame coordinate phantom (30) and the subject (40A) on an image and a positional relationship between said frame and said another frame (20', 20) and the subject (40A) on a real space on the basis of a correlation between the phantom image and the subject image; and
obtaining coordinate data by correcting a deviation between coordinates of the subject (40A) on the real space and coordinates of the subject (40A) on the image photographed by said computed tomographic apparatus in relation to the desired point in the subject (40A) on the basis of the positional relationships on the image and the real space (63).

6. A method according to claim 5, characterized in that the coordinate data is calculated on the basis of a correlation between a plurality of corresponding points or lines on the phantom image and the subject image.

7. A method according to claim 5, further comprising the steps of:
holding the phantom image, and obtaining a positional relationship between said frame coordinate phantom (30) and the subject (40A) on an image and a positional relationship between said frame (20') and the subject (40A) on a real space for each different subject on the basis of a correlation between the held phantom image and the subject image; and
obtaining coordinate data by correcting a deviation between coordinates of the subject (40A) on the real space and coordinates of the subject (40A) of an image photographed by said computed tomographic apparatus in relation to the desired point in the subject (40A) on the basis of the positional relationships on the image and the real space.

8. A method according to claim 7, characterized in that the step of holding the phantom image is the step of storing the phantom image as image data in storage means of said computed tomographic apparatus.

9. A method according to claim 7, characterized in that fine adjustment for high-precision photography to be performed by said computed tomographic apparatus is performed prior to photography of a phantom image or a subject image.

10. A method according to claim 9, characterized in that the fine adjustment is control of a reference frequency corresponding a static field uniformity of changing with passage of time in an MRI apparatus.

11. A method according to claim 7, characterized in that photography conditions for the phantom image are the same as those for the subject image.

12. A method of calculating coordinate data of a desired point in a subject (40A) to be examined by using said apparatus of claim 4, comprising the steps of:
    arranging said another frame (20) having said frame coordinate phantom (30) fixed to an inner surface thereof and said another indicator phantom ($21A_{1'}$, $21A_{2'}$, $21B_{1'}$, $21B_{2'}$) fixed on an outer surface thereof in a photographable region in a computed tomographic apparatus, and integrally photographing said another frame (20), said frame coordinate phantom (30), and said another indicator phantom ($21A_{1'}$, $21A_{2'}$, $21B_{1'}$, $21B_{2'}$), thereby obtaining a phantom image (61);
    arranging said frame (20') holding the subject (40A) therein and having said indicator phantom ($21A_{1'}$, $21A_{2'}$, $21B_{1'}$, $21B_{2'}$) fixed to an outer surface thereof at the same position (62) as that assumed when the phantom image is photographed, and integrally photographing said frame (20'), the subject, and said indicator phantom ($21A_{1'}$, $21A_{2'}$, $21B_{1'}$, $21B_{2'}$), thereby obtaining a subject image;
    obtaining a positional relationship between said frame coordinate phantom (30) and the subject on an image and a positional relationship between said frame and another frame (20', 20) and the subject (40A) on a real space on the basis of a correlation between said another indicator phantom ($21A_{1'}$, $21A_{2'}$, $21B_{1'}$, $21B_{2'}$) on the phantom image and said indicator phantom ($21A_{1'}$, $21A_{2'}$, $21B_{1'}$, $21B_{2'}$) on the subject image (63); and
    obtaining coordinate data by correcting a deviation between coordinates of the subject (40A) on the real space and coordinates of the subject (40A) on the image photographed by said computed tomographic apparatus in relation to the desired point in the subject (40A) on the basis of the positional relationships on the image and the real space.

13. A method according to claim 12, characterized in that the coordinate data is calculated on the basis of a correlation between a plurality of corresponding points or lines on the phantom image and the subject image.

14. A method according to claim 12, further comprising the steps of:
    holding the phantom image, and obtaining a positional relationship between said frame coordinate phantom (30) and the subject (40A) on an image and a positional relationship between said first and second frames (20', 20) and the subject (40A) on a real space for each different subject on the basis of a correlation between said another indicator phantom ($21A_{1'}$, $21A_{2'}$, $21B_{1'}$, $21B_{2'}$) on the held phantom image and said indicator phantom ($21A_{1'}$, $21A_{2'}$, $21B_{1'}$, $21B_{2'}$) on the subject image, and
    obtaining coordinate data by correcting a deviation between coordinates of the subject (40A) on the real space and coordinates of the subject (40A) of an image photographed by said computed tomographic apparatus in relation to the desired point in the subject on the basis of a positional relationship on the image and the real space.

15. A method according to claim 14, characterized in that the step of holding the phantom image is the step of storing the phantom image as image data in storage means of said computed tomographic apparatus.

16. A method according to claim 12, characterized in that fine adjustment for high-precision photography to be performed by said computed tomographic apparatus is performed prior to photography of a phantom image or a subject image.

**17.** A method according to claim 12, characterized in that the fine adjustment is control of a reference frequency corresponding a static field intensity of changing with passage of time in an MRI apparatus.

**18.** A method according to claim 12, characterized in that photography conditions for the phantom image are the same as those for the subject image.

**19.** An apparatus for calculating coordinate data of a desired point in a subject (40A) to be examined by using a frame (20) which has a shape capable of holding the subject (40A) therein and can be arranged in a photographable region in a gantry of a computed tomographic apparatus,

characterized by comprising:

means (22, 22A) for detachably mounting a stereotaxy apparatus on said frame (20) for placement in said gantry;

an indicator phantom (21) affixed outside said frame (20), said indicator phantom (21) comprising means (21A) representing a coordinate arrangement;

a frame coordinate phantom (30) detachably mounted inside said frame (20) in place of said subject (40A);

imaging means (112) for imaging said frame (20) with said subject (40A) and said indicator phantom (21) by said tomographic apparatus to produce a first image (AX), and for imaging said frame (20) with said frame coordinate phantom (30) and said indicator phantom (21) by said tomographic apparatus to produce a second image (AX'); and

calculating means (111) for calculating the coordinate data of the desired point in the subject (40A) based upon a comparison of said first image (AX) and said second image (AX') to obtain calibration data for calibrating a deviation between the position of said frame (20) and the position of the subject (40A).

**20.** Apparatus of claim 19, wherein said frame coordinate phantom (30) comprises marking means such that said second image displays coordinates of at least one axis of X-, Y-, and Z-coordinates.

**21.** The apparatus of claim 19, wherein said indicator phantom (30) comprises marking means such that said first and second images each display coordinates of at least one axis of X-, Y-, and Z-coordinates.

**Patentansprüche**

**1.** Vorrichtung zum Berechnen von Koordinatendaten eines gewünschten Punktes in einem zu untersuchenden Objekt (40A) unter Einsatz eines Rahmens (20'), der eine Form hat, die das Halten des Objektes (40A) im Inneren ermöglicht, und der in einem fotografierbaren Bereich in einem Stützgerüst eines Computertomographs angeordnet werden kann,

**gekennzeichnet durch:**

einen weiteren Rahmen (20), der die gleiche geometrische Gestalt wie der Rahmen (20') hat; und

ein Rahmen-Koordinatenphantom (30), das im Inneren des weiteren Rahmens (20) befestigt ist, ein Koordinatenanzeigeelement, das zum Anzeigen von Koordinaten von zumindest einer Achse von X-, Y- und Z-Koordinaten imstande ist, besitzt und das durch den Computertomographen fotografierbar ist.

**2.** Vorrichtung nach Anspruch 1, dadurch **gekennzeichnet**, daß der Computertomograph ein Magnetresonanz-Abbildungsgerät ist.

**3.** Vorrichtung nach Anspruch 1, dadurch **gekennzeichnet**, daß der Rahmen (20') das abnehmbare Anbringen eines stereotaktischen Geräts (22) an ihm erlaubt.

**4.** Vorrichtung nach Anspruch 1, 2 oder 3, **gekennzeichnet** durch ein Anzeigerphantom (21A$_1$', 21A$_2$', 21B$_1$', 21B$_2$'), das an einer Außenfläche des Rahmens (20') befestigt ist, ein Koordinateranzeigeelement, das zum Anzeigen von Koordinaten von zumindest einer Achse der X-, Y- und Z-Koordinaten imstande ist, aufweist und das durch den Computertomograph fotografierbar ist, und

ein weiteres Anzeigerphantom (21A$_1$', 21A$_2$', 21B$_1$', 21B$_2$'), das an einer Außenfläche eines zweiten Rahmens (20) befestigt ist, ein Koordinatenanzeigeelement, das zum Anzeigen von Koordinaten von mindestens einer Achse der X-, Y- und Z-Koordinaten imstande ist, aufweist, und das durch den Computertomographen fotografierbar ist.

**5.** Verfahren zum Berechnen von Koordinatendaten eines gewünschten Punkts in einem zu untersuchenden Objekt unter Einsatz der Vorrichtung gemäß Anspruch 1, mit dem Schritten:

Anordnen des weiteren Rahmens (20), der das an ihm befestigte Rahmen-Koordinatenphantom (30) aufweist, in einem fotografierbaren Bereich in einem Computertomographen und integrales Fotografieren des weiteren Rahmens (20) und des Rahmen-Koordinatenphantoms (30), wodurch ein Phantombild (61) erhalten wird;

Anordnen des Rahmens (20'), der das in ihm befindliche Objekt hält, an der gleichen Position wie diejenige, die beim Fotografieren des Phantombilds eingenommen wurde, und integrales Fotografieren des Rahmens (20') und des Objekts (40A), wodurch ein Objektbild (62) erhalten wird;

Ermitteln einer positionsmäßigen Beziehung zwischen dem Rahmen-Koordinatenphantom (30) und dem Objekt (40A) auf einem Bild und einer positionsmäßigen Beziehung zwischen dem Rahmen und dem weiteren Rahmen (20', 20) und dem Objekt (40A) in einem reellen Raum auf der Grundlage einer Korrelation zwischen dem Phantombild und dem Objekt; und

Bilden von Koordinatendaten unter Korrektur einer Abweichung zwischen Koordinaten des Objekts (40A) im reellen Raum und von Koordinaten des Objekts (40A) in dem durch den Computertomographen fotografierten Bild bezüglich des gewünschten Punkts in dem Objekt (40A) auf der Grundlage der positionsmäßigen Beziehungen im Bild und im reellen Raum (63).

6. Verfahren nach Anspruch 5, dadurch **gekennzeichnet**, daß die Koordinatendaten auf der Grundlage einer Korrelation zwischen einer Mehrzahl von entsprechenden Punkten oder Linien in dem Phantombild und dem Objektbild berechnet werden.

7. Verfahren nach Anspruch 5, mit den weiteren Schritten:

Beibehalten des Phantombilds und Ermitteln einer positionsmäßigen Beziehung zwischen dem Rahmen-Koordinatenphantom (30) und dem Objekt (40A) in einem Bild sowie einer positionsmäßigen Beziehung zwischen dem Rahmen (20') und dem Objekt (40A) in einem reellen Raum für jedes unterschiedliche Objekt auf der Grundlage einer Korrelation zwischen dem beibehaltenen Phantombild und dem Objektbild; und

Bilden von Koordinaten durch Korrektur einer Abweichung zwischen Koordinaten des Objekts (40A) in dem reellen Raum und den Koordinaten des Objekts (40A) in einem durch den Computertomographen fotografierten Bild bezüglich des gewünschten Bilds in dem Objekt (40A) auf der Grundlage der positionsmäßigen Beziehungen in dem Bild und dem reellen Raum.

8. Verfahren nach Anspruch 7, dadurch **gekennzeichnet**, daß der Schritt des Beibehaltens des Phantombilds ein Schritt des Speicherns des Phantombilds als Bilddaten in einer Speichereinrichtung des Computertomographen ist.

9. Verfahren nach Anspruch 7, dadurch **gekennzeichnet**, daß eine Feineinstellung für eine durch den Computertomographen durchzuführende hochgenaue Fotografie vor der Aufnahme eines Phantombilds oder eines Objektbilds durchgeführt wird.

10. Verfahren nach Anspruch 9, dadurch **gekennzeichnet**, daß die Feineinstellung eine Steuerung einer Referenzfrequenz ist, die einer statischen Feldgleichförmigkeit der zeitlichen Veränderung in einem MRI-Gerät entspricht.

11. Verfahren nach Anspruch 7, dadurch **gekennzeichnet**, daß die Aufnahmebedingungen für das Phantombild die gleichen wie diejenigen für das Objektbild sind.

12. Verfahren zum Berechnen von Koordinatendaten eines gewünschten Punkts in einem zu untersuchenden Objekt (40A) unter Einsatz der Vorrichtung gemäß Patentanspruch 1, mit den Schritten:

Anordnen des weiteren Rahmens (20), der das an einer Innenfläche befestigte Rahmenkoordinatenphantom (30) und das weitere, an einer seiner Außenflächen befestigte Anzeigerphantom ($21A_{1'}$, $21A_{2'}$, $21B_{1'}$, $21B_{2'}$) trägt, in einem fotografierbaren Bereich in einem Computertomographen und integrales Aufnehmen des weiteren Rahmens (20), des Rahmen-Koordinatenphantoms (30) und des weiteren Anzeigerphantoms ($21A_{1'}$, $21A_{2'}$, $21B_{1'}$, $21B_{2'}$), wodurch ein Phantombild (61) erhalten wird;

Anordnen des Rahmens (20'), der das Objekt (40A) in ihm trägt und an dessen einer Außenfläche das Anzeigephantom ($21A_{1'}$, $21A_{2'}$, $21B_{1'}$, $21B_{2'}$) befestigt ist, an der gleichen Position (62) wie diejenige, die beim Aufnehmen des Phantombilds eingenommen wurde, und integrales Aufnehmen des Rahmens (20'), des Objekts und des Anzeigerphantoms ($21A_{1'}$, $21A_{2'}$, $21B_{1'}$, $21B_{2'}$), wodurch ein Objektbild erhalten wird;

Ermitteln einer positionsmäßigen Beziehung zwischen dem Rahmen-Koordinatenphantom (30) und dem Objekt in einem Bild und einer positionsmäßigen Beziehung zwischen dem Rahmen und dem weiteren Rahmen (20', 20) und des Objekts (40A) in einem reellen Raum auf der Grundlage einer Korrelation zwischen dem weiteren Anzeigerphantom ($21A_{1'}$,

21A$_{2'}$, 21B$_{1'}$, 21B$_{2'}$) in dem Phantombild des Anzeigerphantoms (21A$_{1'}$, 21A$_{2'}$, 21B$_{1'}$, 21B$_{2'}$) und dem Objektbild (63); und

Ermitteln von Koordinatendaten durch Korrektur einer Abweichung zwischen Koordinaten des Objekts (40A) in dem reellen Raum und Koordinaten des Objekts (40A) in dem durch den Computertomographen aufgenommenen Bild bezüglich des gewünschten Bilds im Objekt (40A) auf der Grundlage der positionsmäßigen Beziehungen in dem Bild und dem reellen Raum.

13. Verfahren nach Anspruch 12, dadurch **gekennzeichnet**, daß die Koordinatendaten auf der Grundlage einer Korrelation zwischen einer Mehrzahl von entsprechenden Punkten oder Linien in dem Phantombild und dem Objektbild berechnet werden.

14. Verfahren nach Anspruch 12, mit den weiteren Schritten:

Beibehalten des Phantombilds und Ermitteln einer positionsmäßigen Beziehung zwischen dem Rahmen-Koordinatenphantom (30) und dem Objekt (40A) in einem Bild und einer positionsmäßigen Beziehung zwischen dem ersten und dem zweiten Rahmen (20', 20) und dem Objekt (40A) in einem reellen Raum für jedes unterschiedliche Objekt auf der Grundlage einer Korrelation zwischen dem weiteren Anzeigerphantom (21A$_{1'}$, 21A$_{2'}$, 21B$_{1'}$, 21B$_{2'}$) in dem beibehaltenen Phantombild und des Anzeigerphantoms (21A$_{1'}$, 21A$_{2'}$, 21B$_{1'}$, 21B$_{2'}$) in dem Objektbild, und

Ermitteln von Koordinatendaten durch Korrektur einer Abweichung zwischen Koordinaten des Objekts (40A) in dem reellen Raum und Koordinaten des Objekts (40A) in einem durch den Computertomographen fotografierten Bild in Bezug zu dem gewünschten Punkt in dem Objekt auf der Grundlage einer positionsmäßigen Beziehung in dem Bild und dem reellen Raum.

15. Verfahren nach Anspruch 14, dadurch **gekennzeichnet**, daß der Schritt des Beibehaltens des Phantombilds ein Schritt des Speicherns des Phantombilds als Bilddaten in einer Speichereinrichtung des Computertomographen ist.

16. Verfahren nach Anspruch 12, dadurch **gekennzeichnet**, daß eine Feineinstellung für eine durch den Computertomographen durchzuführrerde hochpräzise Aufnahme vor der Aufnahme des Phantombilds oder eines Objektbilds durchgeführt wird.

17. Verfahren nach Anspruch 12, dadurch **gekennzeichnet**, daß die Feineinstellung eine Steuerung einer Referenzfrequenz ist, die einer statischen Feldintensität mit zeitlicher Veränderung in einem MRI-Gerät entspricht.

18. Verfahren nach Anspruch 12, dadurch **gekennzeichnet**, daß Aufnahmebedingungen für das Phantombild die gleichen wie diejenigen für das Objektbild sind.

19. Vorrichtung mm Berechnen von Koordinatendaten eines gewünschten Punkts in einem zu untersuchenden Objekt (40A) unter Einsatz eines Rahmens (20), der eine zum Halten eines Objekts (40A) in ihm geeignete Gestalt besitzt und in einem fotografierbaren Bereich in einem Stützgerüst eines Computertomographen angeordnet werden kann,

**gekennzeichnet** durch

eine Einrichtung (22, 22A) mm abnehmbaren Anbringen eines Stereotaxiegeräts an dem Rahmen (20) für die Anordnung in dem Stützgerüst;

ein Anzeigerphantom (21), das außerhalb bzw. an der Außenseite des Rahmens (20) befestigt ist, wobei das Anzeigerphantom (21) eine eine Koordinatenanordnung repräsentierende Einrichtung (21A) aufweist;

ein Rahmen-Koordinatenphantom (30), das abnehmbar im Inneren des Rahmens (20) anstelle des Objekts (40A) angebracht ist;

eine Abbildungseinrichtung (112) zum Abbilden des Rahmens (20) mit dem Objekt (40A) und dem Anzeigerphantom (21) durch den Tomograph zum Erzeugen eines ersten Bilds (AX) sowie zum Abbilden des Rahmens (20) mit dem Rahmen-Koordinatenphantom (30) und dem Anzeigerphantom (21) durch den Tomograph zur Erzeugung eines zweiten Bilds (AX');

eine Berechnungseinrichtung (111) zum Berechnen der Koordinatendaten des gewünschten Punkts in dem Objekt (40A) auf der Grundlage eines Vergleichs des ersten Bilds (AX) und des zweiten Bilds (AX') zur Bildung von Kalibrierungsdaten für die Kalibrierung einer Abweichung zwischen der Position des Rahmens (20) und der Position des Objekts (40A).

20. Vorrichtung nach Anspruch 19, bei dem das Rahmen-Koordinatenphantom (30) eine Markierungseinrichtung derart aufweist, daß das zweite Bild Koordinaten von zumindest einer Achse der X-, Y- und Z-Koordinaten anzeigt.

**21.** Vorrichtung nach Anspruch 19, dadurch **gekennzeichnet**, daß das Anzeigerphantom (30) eine Markierungseinrichtung derart aufweist, daß das erste und das zweite Bild jeweils Koordinaten von zumindest einer Achse der X-, Y- und Z-Koordinaten anzeigen.

## Revendications

**1.** Appareil de calcul de données de coordonnées d'un point voulu dans un sujet (40A) à examiner à l'aide d'un cadre (20') ayant une configuration permettant le maintien du sujet (40A) à l'intérieur et qui peut être placé dans une région qui peut être photographiée dans un portique d'un appareil de tacographie, caractérisé en ce qu'il comprend

un autre cadre (20) ayant géométriquement la même configuration que ledit cadre (20'), et

un fantôme (30) de coordonnées du cadre qui est fixé à l'intérieur de l'autre cadre (20), qui a un organe d'affichage de coordonnées qui peut afficher les coordonnées suivant au moins l'un des axes de coordonnées X, Y et Z et qui peut être photographié par l'appareil de tacographie.

**2.** Appareil selon la revendication 1, caractérisé en ce que l'appareil de tacographie est un appareil de formation d'images par résonance magnétique.

**3.** Appareil selon la revendication 1, caractérisé en ce que ledit cadre (20') permet un montage amovible sur lui d'un appareil stéréotaxique (22).

**4.** Appareil selon la revendication 1, 2 ou 3, comprenant un fantôme indicateur ($21A_1'$, $21A_2'$, $21B_1'$, $21B_2'$), qui est fixé à une surface externe du cadre (20'), et ayant un organe d'affichage de coordonnées qui peut afficher les coordonnées d'au moins un axe parmi les axes de coordonnées X, Y et Z, et qui peut être photographié par l'appareil de tacographie, et

un autre fantôme indicateur ($21A_1'$, $21A_2'$, $21B_1'$, $21B_2'$) qui est fixé à une surface externe de l'autre cadre (20), qui possède un organe d'affichage de coordonnées capable d'indiquer les coordonnées suivant au moins un axe des coordonnées X, Y et Z, et qui peut être photographié par l'appareil de tacographie.

**5.** Procédé de calcul de données de coordonnées d'un point voulu dans un sujet à examiner à l'aide de l'appareil selon la revendication 1, comprenant les étapes suivantes :

la disposition de l'autre cadre (20) ayant le fantôme (30) de coordonnées de cadre qui lui est fixé dans une région qui peut être photographiée dans un appareil de tacographie, et la photographie complète de l'autre cadre (20) et du fantôme (30) de coordonnées de cadre avec obtention d'une image de fantôme (61),

la disposition du cadre (20') maintenant le sujet à l'intérieur à la position prise antérieurement lors de la photographie de l'image du fantôme, et la photographie complète du cadre (20') et du sujet (40A) avec obtention d'une image (62) du sujet,

l'obtention d'une relation de position entre le fantôme (30) de coordonnées du cadre et le sujet (40A) sur une image et d'une relation de position entre le cadre et l'autre cadre (20', 20) et le sujet (40A) dans un espace réel sur la base d'une corrélation entre l'image du fantôme et l'image du sujet, et

l'obtention de données de coordonnées par correction d'un écart entre les coordonnées du sujet (40A) dans l'espace réel et les coordonnées du sujet (40A) sur l'image photographiée par l'appareil de tacographie par rapport au point voulu dans le sujet (40A) en fonction des relations de position sur l'image et dans l'espace réel (63).

**6.** Procédé selon la revendication 5, caractérisé en ce que les données de coordonnées sont calculées sur la base d'une corrélation entre plusieurs points ou lignes correspondants sur l'image du fantôme et l'image du sujet.

**7.** Procédé selon la revendication 5, comprenant en outre les étapes suivantes :

la conservation de l'image du fantôme et l'obtention d'une relation de position entre le fantôme (30) de coordonnées du cadre et le sujet (40A) sur une image et d'une relation de position entre le cadre (20') et le sujet (40A) dans un espace réel pour chaque sujet différent d'après la corrélation entre l'image du fantôme et l'image du sujet, et

l'obtention de données de coordonnées par correction d'un écart entre les coordonnées du sujet (40A) dans l'espace réel et les coordonnées du sujet (40A) d'une image photographiée par l'appareil de tacographie par rapport au point voulu dans le sujet (40A) en fonction des relations de position sur l'image et dans l'espace réel.

**8.** Procédé selon la revendication 7, caractérisé en ce que l'étape de conservation de l'image du fantôme est une étape de mémorisation de l'image du fantôme sous forme de données d'image dans un dispositif de mémorisation de l'appareil de tacographie.

9. Procédé selon la revendication 7, caractérisé en ce que l'ajustement fin nécessaire à une photographie de haute précision qui doit être exécutée par l'appareil de tacographie est réalisée avant la photographie d'une image du fantôme ou d'une image du sujet.

10. Procédé selon la revendication 9, caractérisé en ce que l'ajustement fin est le réglage d'une fréquence de référence correspondant à une uniformité du champ statique de changement au cours du temps dans un appareil d'imagerie IRM.

11. Procédé selon la revendication 7, caractérisé en ce que les conditions de photographie de l'image du fantôme sont les mêmes que celles de l'image du sujet.

12. Procédé de calcul de données de coordonnées d'un point voulu dans un sujet (40A) à examiner par utilisation de l'appareil selon la revendication 4, comprenant les étapes suivantes :
la disposition de l'autre cadre (20) ayant le fantôme (30) de coordonnées de cadre fixé à une surface interne et de l'autre fantôme indicateur ($21A_1'$, $21A_2'$, $21B_1'$, $21B_2'$) fixé à la surface externe dans une région qui peut être photographiée dans un appareil de tacographie, et la photographie complète de l'autre cadre (20), du fantôme (30) de coordonnées du cadre et de l'autre fantôme indicateur ($21A_1'$, $21A_2'$, $21B_1'$, $21B_2'$) avec obtention d'une image du fantôme (61),
la disposition du cadre (20') portant le sujet (40A) à l'intérieur et ayant le fantôme indicateur ($21A_1'$, $21A_2'$, $21B_1'$, $21B_2'$) fixé à une surface externe à la position (62) déjà prise lorsque l'image du fantôme est photographiée, et la photographie complète du cadre (20'), du sujet et du fantôme indicateur ($21A_1'$, $21A_2'$, $21B_1'$, $21B_2'$) avec obtention de cette manière d'une image du sujet,
l'obtention d'une relation de position entre le fantôme (30) de coordonnées du cadre et le sujet sur une image et d'une relation de position entre le cadre et l'autre cadre (20', 20) et le sujet (40A) dans l'espace réel en fonction d'une corrélation entre l'autre fantôme indicateur ($21A_1'$, $21A_2'$, $21B_1'$, $21B_2'$) sur l'image du fantôme et le fantôme indicateur ($21A_1'$, $21A_2'$, $21B_1'$, $21B_2'$) sur l'image du sujet (63), et
l'obtention de données de coordonnées par correction d'un écart entre les coordonnées du sujet (40A) dans l'espace réel et les coordonnées du sujet (40A) sur l'image photographiée par l'appareil de tacographie par rapport au point voulu dans le sujet (40A) en fonction des relations de position sur l'image et dans l'espace réel.

13. Procédé selon la revendication 12, caractérisé en ce que les données de coordonnées sont calculées d'après une corrélation entre plusieurs points ou lignes correspondants sur l'image du fantôme et l'image du sujet.

14. Procédé selon la revendication 12, comprenant en outre les étapes suivantes :
la conservation de l'image du fantôme et l'obtention d'une relation de position entre le fantôme (30) des coordonnées du cadre et le sujet (40A) sur une image et d'une relation de position entre le premier et le second cadre (20', 20) et le sujet (40A) dans l'espace réel pour chaque sujet différent en fonction d'une corrélation entre l'autre fantôme indicateur ($21A_1'$, $21A_2'$, $21B_1'$, $21B_2'$) sur l'image conservée du fantôme et le fantôme indicateur ($21A_1'$, $21A_2'$, $21B_1'$, $21B_2'$) sur l'image du sujet, et
l'obtention de données de coordonnées par correction d'un écart entre les coordonnées du sujet (40A) dans l'espace réel et les coordonnées du sujet (40A) sur une image photographiée par l'appareil de tacographie par rapport au point voulu dans le sujet en fonction d'une relation de position sur l'image et dans l'espace réel.

15. Procédé selon la revendication 14, caractérisé en ce que l'étape de conservation de l'image du fantôme est une étape de mémorisation de l'image du fantôme sous forme de données d'image dans le dispositif de mémorisation de l'appareil de tacographie.

16. Procédé selon la revendication 12, caractérisé en ce que l'ajustement fin qui doit être réalisé pour la photographie de haute précision par l'appareil de tacographie est réalisé avant la photographie d'une image de fantôme ou d'une image d'un sujet.

17. Procédé selon la revendication 12, caractérisé en ce que l'ajustement fin est un réglage d'une fréquence de référence correspondant à une intensité en champ statique de changement au cours du temps dans un appareil IRM.

18. Procédé selon la revendication 12, caractérisé en ce que les conditions de photographie de l'image du fantôme sont les mêmes que celles de l'image du sujet.

19. Appareil de calcul de données de coordonnées d'un point voulu dans un sujet (40A) à examiner à l'aide d'un cadre (20) ayant une configuration permettant la retenue du sujet (40A) à l'intérieur et qui peut être placé dans une région qui peut être photographiée dans un portique d'un appareil de tacographie,

caractérisé par

un dispositif (22, 22A) de montage temporaire d'un appareil de prise de vues stéréotaxiques sur le cadre (20), pour la disposition dans le portique,

un fantôme indicateur (21) fixé à l'extérieur du cadre (20), le fantôme indicateur (21) comprenant un dispositif (21A) qui représente un ensemble de coordonnées,

un fantôme (30) de coordonnées de cadre monté de façon temporaire à l'intérieur du cadre (20) à la place du sujet (40A),

un dispositif (112) de formation d'une image du cadre (20) avec le sujet (40A) et le fantôme indicateur (21) par l'appareil de tacographie pour la création d'une première image (AX), et pour la formation d'une image du cadre (20) avec le fantôme (30) de coordonnées du cadre et le fantôme indicateur (21) à l'aide de l'appareil de tacographie pour la production d'une seconde image (AX'), et

un dispositif (111) de calcul des données de coordonnées du point voulu dans le sujet (40A) d'après une comparaison de la première image (AX) et de la seconde image (AX') pour l'obtention de données d'étalonnage d'un écart entre la position du cadre (20) et la position du sujet (40A).

20. Appareil selon la revendication 19, dans lequel le fantôme (30) de coordonnées comporte un dispositif de marquage tel que la seconde image affiche les coordonnées d'au moins un axe parmi les axes de coordonnées X, Y et Z.

21. Appareil selon la revendication 19, dans lequel le fantôme indicateur (30) comporte un dispositif de marquage tel que la première et la seconde image affichent chacune des coordonnées d'au moins un axe parmi les axes de coordonnées X, Y et Z.

EP 0 422 396 B1

F I G. 1

F I G. 2

16

STATIC FIELD CONTROL SYSTEM

104

101

101

102

103

105 X-AXIS GRADIENT FIELD POWER SOURCE

106 Y-AXIS GRADIENT FIELD POWER SOURCE

107 Z-AXIS GRADIENT FIELD POWER SOURCE

108 TRANSMITTER

109 RECEIVER

110 SEQUENCER

111 COMPUTER SYSTEM

114 MEMORY

112 DISPLAY SYSTEM

F I G. 3

F I G. 4

F I G. 5

F I G. 6

F I G. 7

F I G. 8

EP 0 422 396 B1

F I G.  9A

F I G.  9B

F I G.  10

F I G.  11

F I G.  12

F I G.  13

21

FIG. 14A

FIG. 14B

FIG. 15A

FIG. 15B

F I G. 16

F I G 17

F I G. 18

F I G. 19

F I G. 20

F I G. 21

F I G. 22

EP 0 422 396 B1

F I G. 23A

21B₁

20

21A₂

ηP

30

ξP

21B₂

21A₁

F I G. 23B

23

20'

21A'₂

21B'₁

23

21B'₂

21A'₁

25

23

23

20

$\eta^P$

30

$\xi^P$

F I G. 24A

23

23

20

F I G. 24B

20

$\eta^P$

30

$\xi^P$

F I G. 25A

23

23

20′

F I G. 25B

PHOTOGRAPH PHANTOM IMAGES BY USING FRAME HAVING FRAME COORDINATE PHANTOM MOUNTED THEREON ~ 51

DETACH FRAME COORDINATE PHANTOM FROM FRAME AND HOLD SUBJECT INSTEAD ~ 52

PHOTOGRAPH SUBJECT IMAGE BY USING FRAME HOLDING SUBJECT ~ 53

OBTAIN CORRECTED COORDINATE DATA OF SPECIFIC POINT ON THE BASIS OF PHANTOM IMAGE AND SUBJECT IMAGE ~ 54

DETACH INDICATOR PHANTOMS FROM FRAME AND MOUNT STEREOTATIC APPARATUS INSTEAD ~ 55

OPERATE STEREOTATIC APPARATUS ON THE BASIS OF OBTAINED COORDINATE DATA TO PERFORM SURGICAL OPERATION ~ 56

F I G. 26

| | |
|---|---|
| PHOTOGRAPH PHANTOM IMAGES BY USING SECOND FRAME HAVING FRAME COORDINATE PHANTOM MOUNTED THEREON | 61 |

| | |
|---|---|
| PHOTOGRAPH SUBJECT IMAGE BY USING FIRST FRAME HOLDING SUBJECT | 62 |

| | |
|---|---|
| OBTAIN CORRECTED COORDINATE DATA OF SPECIFIC POINT ON THE BASIS OF PHANTOM IMAGE AND SUBJECT IMAGE | 63 |

| | |
|---|---|
| DETACH INDICATOR PHANTOMS FROM FIRST FRAME AND MOUNT STEREOTATIC APPARATUS INSTEAD | 64 |

| | |
|---|---|
| OPERATE STEREOTATIC APPARATUS ON THE BASIS OF COORDINATE DATA TO PERFORM SURGICAL OPERATION | 65 |

F I G. 27